# EUROPÄISCHE PATENTSCHRIFT

(11) **EP 1 402 917 B1**
(45) Veröffentlichungstag und Bekanntmachung des Hinweises auf die Patenterteilung: **02.03.2011**
(21) Anmeldenummer: 03103261.8
(22) Anmeldetag: 01.09.2003
(51) Int. Cl.: A61M 25/00, A61B 5/00

(54) **Katheter mit optischer Faser**
Catheter comprising an optical fiber
Cathéter avec fibre optique

(30) Priorität: 28.09.2002 DE 10245416
(43) Veröffentlichungstag der Anmeldung: 31.03.2004
(73) Patentinhaber: Pulsion Medical Systems AG, 81829 München (DE)
(72) Erfinder: Pfeiffer, Ulrich J., Dr., 81667 München (DE)
(74) Vertreter: Kehl, Günther

(56) Entgegenhaltungen:
- EP-A- 0 266 928
- WO-A-85/02101
- DE-A- 4 024 677
- US-A- 5 673 694
- US-A- 5 892 871

## Beschreibung

Die vorliegende Erfindung betrifft Kathetersysteme zur kontinuierlichen Messung der zentralvenösen Sauerstoffsättigung und/oder der Messung der lokalen Konzentration injizierten Indocyaningrüns, insbesondere Kathetersysteme mit einem flexiblen, länglichen, zentralvenös applizierbaren Grundkörper, einer faseroptischen Sonde zur Vornahme reflexionsoximetrischer Messungen, einem Faseroptiklumen zur Aufnahme der faseroptischen Sonde und mit Befestigungsmitteln zur Vermeidung einer Längsverschiebung der faseroptischen Sonde relativ zu dem Faseroptiklumen, wobei die Befestigungsmittel lösbar sind, um eine Längsverschiebung der faseroptischen Sonde relativ zu dem Faseroptiklumen zur Entnahme der faseroptischen Sonde zuzulassen.

Im operativen Bereich und in der Intensivmedizin werden sehr häufig zentralvenöse Katheter (ZVK) mit mehreren Lumina, sogenannte Multilumen-ZVK, gelegt, die dazu dienen
- den zentralvenösen Druck zu messen,
- Infusionslösungen, Blut und Blutderivate sowie Pharmaka gleichzeitig über verschiedene Lumina zuzuführen, und
- Blutproben für blutgasanalytische, hämatologische und biochemische Untersuchungen zu entnehmen.

Im Rahmen der zentralvenösen Blutgasanalyse interessiert dabei insbesondere die zentralvenöse Sauerstoffsättigung (Scv02), da aus ihr wertvolle Hinweise über die Sauerstoffverfügbarkeit und Sauerstoffausschöpfung des Gesamtorganismus gewonnen werden können. Mit Hilfe eines kontinuierlichen Monitoring der zentralvenösen Sauerstoffsättigung mittels eines faseroptischen zentralvenösen Katheters können aus einem Abfall der zentralvenösen Sauerstoffsättigung ein Sinken des Herzzeitvolumens, eine Abnahme des Sauerstoffträgers Hämoglobin, eine verminderte Sauerstoffzufuhr durch die Beatmung oder ein nicht kompensierter Anstieg des Sauerstoffverbrauchs des Organismus schnell erkannt werden. Insofern eignet sich das kontinuierliche Monitoring der zentralvenösen Sauerstoffsättigung als kostengünstige, globale physiologische Überwachungsmethode. Die zentralvenöse Sauerstoffsättigung kann kontinuierlich mittels Faseroptikreflexionsoximetrie bei einer Meßwellenlänge von etwa 660 nm im strömenden Blut gemessen werden. Optische Strahlung einer anderen Wellenlänge von üblicherweise etwa 930 nm dient als sogenannte Referenzwellenlänge. Bei dieser Wellenlänge existiert kein wesentlicher Unterschied zwischen der Reflexion von sauerstoffbeladenem und sauerstofffreiem Hämoglobin. Das Meßergebnis bei der Referenzwellenlänge dient dazu, strömungsbedingte und andere Artefakte zu kompensieren. Bei Anwesenheit des gelegentlich in die Blutbahn eingebrachten Diagnostikums Indocyaningrün können allerdings Meßfehler auftreten, da Indocyaningrün bei 660 nm und 930 nm unterschiedlich stark absorbiert.

Mittels Indocyaningrün (lCG) wird bei vielen kritisch kranken Patienten im operativen Bereich und in der Intensivmedizin ein Leberfunktionstest durchgeführt. Indocyaningrün ist ein gut verträgliches Pharmakon, das sich nach intravenöser Applikation sofort an Plasmaproteine bindet, vorwiegend im Blutraum verweilt und ausschließlich von der Leber mit einer normalen Halbwertszeit von 3-4 Minuten unverändert in die Galle ausgeschieden wird. Indocyaningrün kann mittels Faseroptikreflexionsdensitometrie kontinuierlich bei einer Meßwellenlänge von etwa 805 nm gemessen werden. Hier kann optische Strahlung bei etwa 900 nm als Referenzwellenlänge dienen. Insbesondere bei kritisch kranken, hämodynamisch instabilen Patienten wäre es besonders wichtig, auch während der Leberfunktionsbestimmung mittels Indocyaningrün die kontinuierliche Messung der zentralvenösen Sauerstoffsättigung durchführen zu können.

Die Druckschrift DE 4024677 zeigt die Merkmale des Oberbegriffs von Anspruch 1.

Ein Kathetersystem zur kontinuierlichen Messung der zentralvenösen Sauerstoffsättigung, bei welchem jedoch im Unterschied zu Kathetersystemen der eingangs genannten Art die Faseroptik permanent in einem Multilumen-ZVK fixiert ist, ist aus der US-Patentschrift 5,315,995 bekannt. Das faseroptische Bündel, welches zur Messung der zentralvenösen Sauerstoffsättigung dient, endet direkt am distalen Ende des Katheters. Dabei weist das distale Ende eine plane Fläche auf, der Katheter ist sozusagen in Bezug auf den Katheterschaft rechtwinklig abgeschnitten. Die relativ große distale Fläche bedingt, daß der Katheter mangels sich verjüngender Spitze nicht mittels der sogenannten Seldinger-Technik plaziert werden kann. Darunter versteht man ein Verfahren, bei welchem - nach Punktion des Blutgefäßes, in das ein Katheter eingeführt werden soll - über die Punktionskanüle ein dünner Führungsdraht mit in der Regel etwa der doppelten Länge des distalen Lumens (= Kanal, dessen Ende am weitesten vom Untersucher entfernt ist), das zentral in Bezug auf den runden Querschnitt an der Spitze des Katheters endet, in das Blutgefäß vorgeschoben wird. Nach Vorschieben des Führungsdrahtes wird die Punktionskanüle durch Zurückziehen über den Draht entfernt. Dann wird ein sogenannter Dilatator, ein relativ robuster und steifer, einlumiger Katheter aus Plastikmaterial mit sich auf den Durchmesser des Führungsdrahtes verjüngender distaler Spitze, über den Führungsdraht in das Gefäß vorgeschoben. Der Dilatator hat die Aufgabe, den Stichkanal durch Haut-, Fett- und Muskelgewebe und die Blutgefäßwandung auf den Durchmesser des Katheters aufzudehnen. Nach Aufdehnung wird der Dilatator entfernt, der Führungsdraht bleibt mit der distalen Spitze im Blutgefäß liegen. Nun wird das freie proximale (= dem Untersucher am nächsten befindliche) Ende des Führungsdrahtes in die ebenfalls auf Führungsdrahtdurchmesser hin verjüngte Spitze des Katheters eingeführt und dieser über den Führungsdraht in das Blutgefäß vorgeschoben. Sobald der Katheter richtig plaziert ist, wird der Führungsdraht aus dem sogenannten distalen Lumen herausgezogen; damit steht das distale Lumen des Katheters für andere Zwecke zur Verfügung.

Stattdessen kann der aus US 5,315,995 vorbekannte Multilumen-ZVK nur durch einen zuvor plazierten Einführungskatheter, einen sogenannten Introducer, in die richtige Position im zentralen Venensystem vorgeschoben werden kann. Dieses Verfahren ist umständlich und zeitaufwendig, außerdem weist der Introducer natürlich einen erheblich größeren Außendurchmesser auf, als der einzuführende Multilumen-ZVK. Deshalb erfolgt hier eine ungünstige und risikoreichere Gefäßpunktion mit größerem Durchmesser, als eigentlich für die Plazierung eines Multilumen-ZVK mit Seldinger-Technik erforderlich wäre. Des weiteren kann das bekannte Kathetersystem nicht eingesetzt werden, die Indocyaningrünkonzentration im Blut zu messen.

Ein Kathetersystem der eingangs genannten Art zur kontinuierlichen Messung der zentralvenösen Sauerstoffsättigung ist aus der US-Patentschrift 5,673,694 bekannt. Darin werden eine Faseroptiksonde und ein Faseroptikkatheter mit durchgehendem, parallel zum Faseroptiklumen verlaufendem Lumen zur kontinuierlichen Spülung der im Bereich der distalen Spitze endenden Faseroptik beschrieben. Das Kathetersystem weist eine flexibel einstellbare Länge des Teiles der Faseroptiksonde auf, der in das distale Lumen des bereits liegenden Multilumen-ZVK eingeführt wird. Da die Faseroptiksonde mittels einer kraftschlüssigen Feststellvorrichtung in der Länge flexibel vorschiebbar ist, muß der außerhalb des Multilumen-ZVK befindliche Teil der Faseroptiksonde bzw. des Faseroptikkatheters mittels eines sterilen Überziehers vor bakterieller Kontamination geschützt werden. Die vorbekannte Vorrichtung hat insbesondere den Nachteil, daß das Faseroptiklumen des Multilumen-ZVK, in das die Faseroptiksonde eingeführt wird, selbst nicht gespült werden kann, und somit die Gefahr der Bildung von Blutgerinnseln an der distalen Austrittsstelle des Faseroptiklumens besteht. Des weiteren kann das bekannte Kathetersystem nicht eingesetzt werden, die Indocyaningrünkonzentration im Blut zu messen.

Angesichts der geschilderten Probleme, welche die Anwendung bekannter Kathetersysteme zur Messung der zentralvenösen Sauerstoffsättigung in der klinischen Routine erheblich erschweren, liegt der vorliegenden Erfindung die Aufgabe zugrunde, ein Kathetersystem der eingangs genannten Art zu schaffen, welches im medizinischen Einsatz größere Sicherheit gegen bakterielle Kontamination sowie eine einfachere Handhabung gewährleistet, als herkömmliche Systeme. Ferner soll der intravenöse Teil des Systems auf einfache, für den Patienten möglichst schonende Weise applizierbar sein. Ferner sollen eventuell gleichzeitig vorzunehmende Scv02- und ICG-Messungen bei Einsatz des Kathetersystems einander nicht negativ beeinflussen.

Gemäß einem Aspekt der Erfindung wird diese Aufgabe durch ein Kathetersystem gemäß Anspruch 1 gelöst. Entgegen dem Stand der Technik ist die Länge des in das Faseroptiklumen eines zentralvenösen Katheters eingeführten Teils der Faseroptiksonde nicht über eine einfache Klemmverbindung frei verschieblich. Die Faseroptik ist zwar herausnehmbar, allerdings ist die Verbindung zwischen Anschlußstück und Gegenstück formschlüssig, was die Handhabung stark vereinfacht und zugleich für die Gestaltung des proximalen Teils des Kathetersystems vielfältigere Möglichkeiten offenhält. Ein zusätzlicher, umständlicher und empfindlicher, überzieherartiger Kontaminationsschutz gemäß dem Stand der Technik kann entfallen.

Bei dem Faseroptiklumen handelt es sich grundsätzlich um ein speziell ausgebildetes, distales oder nahe einem distalen Lumen endendes Lumen zur Aufnahme der einschiebbaren Faseroptiksonde nach Plazierung des Katheter-Grundkörpers im Blutgefäßsystem.

Die flexible Faseroptiksonde ist auf das in dem zentralvenösen Katheter ausgebildete Faseroptiklumen adaptiert; die Länge der Faseroptiksonde ist dabei so bemessen, daß sie nach Plazierung und Arretierung im Faseroptiklumen des zentralvenösen Katheters mit der flexiblen Spitze im strömenden Blut liegt. Dabei kann sie vorteilhafterweise mit der Spitze des Katheter-Grundkörpers plan abschließen. Alternativ hierzu kann sie auch vorzugsweise 10-40 mm über das distale Ende des Faseroptiklumen frei in die Blutströmung hinausragen. Dadurch ist sichergestellt, daß die Faseroptiksonde die üblichwerweise weiche Spitze des Katheter-Grundkörpers nicht versteift - die Faseroptiksonde biegt sich bei Gefäßkontakt somit sehr leicht, was ein erwünschter Effekt ist.

Die Fiberoptiksonde enthält ein distal endendes, vorzugsweise zur Reflexionsoximetrie und Reflexionsdensitometrie für Indocyaningrün geeignetes Faseroptiksystem. Ein vorzugsweise vorgesehener, sehr dünn und flexibel ausgebildeter äußerer Schaft der Faseroptiksponde besteht aus bio- und hämokompatiblem Material, wie z.B. Polyurethan, die darin untergebrachte Faseroptik besteht vorzugsweise aus flexiblen Kunststofffasern mit guten Transmissionseigenschaften für sichtbares und nahinfrarotes Licht, beispielsweise aus Polyacrylamid. Der mit dem Blut in Kontakt kommende distale Endbereich der Faseroptiksonde ist vorzugsweise mit antithrombogenem Überzug, bestehend beispielsweise aus Heparin oder Parylen (Di-para-xylene), versehen, um das Entstehen von Blutgerinseln zu vermeiden.

Gemäß einer besonders bevorzugten Ausführungsform der Erfindung ist das Faseroptiklumen spülbar. Es ist hierfür so groß ausgebildet, daß neben der einschiebbaren Faseroptiksonde genügend Raum bleibt, um das Restlumen mit üblichen Katheterspülvorrichtungen zu spülen, oder aber den zentralvenösen Druck zu messen und auch Blut abzunehmen. Zum Anschluß einer Spülvorrichtung ist vorzugsweise ein in das Faseroptiklumen bzw. dessen Fortsetzung mündender Spülansatz oder Spülstutzen am mit der Faseroptiksonde verbundenen Anschlußstück vorgesehen. Alternativ kann aber auch am entsprechenden Gegenstück des zentralvenösen Katheters eine Anschlußmöglichkeit für eine Spülvorrichtung vorhanden sein.

Vorzugsweise wird das Kathetersystem mit Einführungshilfsmitteln kombiniert. Gemeint sind hiermit "innere" Einführungshilfsmittel zum Setzen des Katheters mittels der oben beschriebenen Seldingertechnik, weniger "äußere" Einführungsmittel, d.h. Einführungskatheter gemäß dem Stand der Technik.

Gemäß einer vorteilhaften Weiterbildung der Erfindung ist das Kathetersystem mit einem zusätzlichen Rechtsherzkatheter (Pulmonalarterienkatheter, Katheter nach Swan und Ganz) kombiniert. Das Kathetersystem weist dann einen Multilumen-ZVK mit entsprechend großem distalem Lumen auf, durch welches ein zusätzlicher Rechtsherzkatheter in das zentrale Venensystem vorgeschoben werden kann. In diesem Falle endet das Faseroptiklumen nahe und parallel zum distalen Lumen. Diese Variante kann zusätzlich einen an der Spitze auf den Durchmesser eines geeigneten Führungsdrahtes sich verjüngenden Einführungs-Hohlmandrin aufweisen, der durch den sich distal auf den Durchmesser des Einführungs-Hohlmandrin's verjüngenden Multilumen-ZVK geschoben wird. Der Einführungs-Hohlmandrin kommt nur zur Plazierung des Multilumen-ZVK zum Einsatz. Der Führungsdraht wird mit seinem proximalen Ende von der distalen Seite her in den durch den Mulitlumen-ZVK geschobenen Einführungs-Hohlmandrin eingeschoben. Der Mulitlumen-ZVK verfügt vorzugsweise zwischen 2 und 5 Lumina und weist - falls er in Richtung vom Kopf zum Herzen über eine große Halsvene oder über eine große Vene unterhalb des Schlüsselbeins plaziert wird - Längen von vorzugsweise 10 bis 30 cm auf. Generell wird die erforderliche Länge so gewählt, daß der betreffende zentralvenöse Multilumenkatheter mit einem ausreichenden Sicherheitsabstand zum Herzen bzw. zum rechten Vorhof endet, um zu vermeiden, daß das Herz bzw. der rechte Vorhof durch die Katheterspitze mechanisch gestört wird, aber auch, um das Risiko einer Perforation des Katheters in herznahen venösen Gefäßstrukturen oder im rechten Vorhof so gering wie möglich zu halten. Das Material des zentralvenösen Multilumenkatheters besteht aus sehr gut blutverträglichen Materialien, vorzugsweise aus Polyurethan, das zusätzlich noch mit antithrombogenen und antimikrobiellen Substanzen beschichtet oder versetzt ist. Ferner weist der Katheter eine Spitze auf, die weicher als der Katheterschaft ist, um das zuvor schon beschriebene Risiko einer Perforation so gering wie möglich zu halten. Was Länge und Material betrifft, kann der Kathetergrundkörper vorteilhafterweise auch bei Ausbildungsformen ohne Kombination mit Rechtsherzkatheter entsprechend ausgebildet sein.

Gemäß einer besonders bevorzugten Ausführungsform der Erfindung ist die Faseroptiksonde zur gleichzeitigen, gegenseitig unbeeinflußten Messung der zentralvenösen Sauerstoffsättigung und der lokalen Konzentration injizierten Indocyaningrüns ausgebildet. Hierzu ist sie vorteilhafterweise an eine Lichtquellen- und Meßvorrichtung anschließbar, welche zur gleichzeitigen Aussendung und Messung von Strahlung zweier Meß- und einer Referenzwellenlänge ausgebildet ist. Vorzugsweise liegt die erste Meßwellenlänge bei 660 nm, die zweite Meßwellenlänge bei 805 nm, und die Referenzwellenlänge bei 880 nm. Im Wellenlängenbereich von 660 nm unterscheiden sich die Reflexionseigenschaften von sauerstoffbeladenem und sauerstofffreiem Hämoglobin im Blut besonders stark. Bei 805 nm liegt das Absorptionsmaximum von Indocyaningrün. Die Referenzwellenlänge von 880 nm hat sich als besonders günstig herausgestellt, da hier kein wesentlicher Unterschied der Reflexion zwischen sauerstoffgeladenem und sauerstofffreiem Hämoglobin im Blut existiert, und Indocyaningrün denselben molaren Absorptions- bzw. Reflexionskoeffizienten aufweist, wie oxygeniertes Hämoglobin. Auch wenn die genannten Wellenlängen besonders günstig sind, können insbesondere auch Wellenlängen von jeweils bis zu 10 nm (unter Umständen auch mehr) über bzw. unter den genannten Werten als erfindungsgemäß gelten, wobei hier größere Meßfehler auftreten könnten. Vorteilhafterweise ist die Lichtquellen- und Meßvorrichtung mit einer Auswerteinheit funktional zusammengeschlossen, welche mit Mitteln zur Berechnung der zentralvenösen Sauerstoffsättigung anhand des Verhältnisses zwischen Reflexionsmessungen bei der ersten Meßwellenlänge und der Referenzwellenlänge, sowie mit Mitteln zur Berechnung der zentralvenösen Konzentration von Indocyaningrün anhand des Verhältnisses zwischen Reflexionsmessungen bei der zweiten Meßwellenlänge und der Referenzwellenlänge ausgestattet. Alle Mittel zur Berechnung sind in aller Regel computerimplementiert und können auf aus Literatur und Technik bekannten Algorithmen aufbauen.

Gemäß einer weiteren vorteilhaften Weiterbildung der Erfindung kann das Kathetersystem zusätzlich mit Heizmitteln wie in EP 1 236 435 A1 offenbart ausgestattet sein.

Nachfolgend wird ein erfindungsgemäßes Ausführungsbeispiel anhand der zugehörigen Zeichnungen beschrieben. Die Abbildungen sind hierbei nicht maßstabsgetreu und schematisch. Dargestellt ist in
- Fig. 1a: eine mehrfach unterbrochene Teilansicht eines zentralvenösen Multilumenkatheters, welcher Teil eines erfindungsgemäßen Kathetersystems ist; in
- Fig. 1b: eine Ansicht einer erfindungsgemäßen faseroptischen Sonde, welche an eine erfindungsgemäße Lichtquellen- und Meßvorrichtung angeschlossen ist; und in
- Fig. 2: eine mittels gestricheltem Kreis K in Fig. 1b angedeutete Teilansicht im Querschnitt, welche im wesentlichen das fest mit der faseroptischen Sonde verbundene Anschlußstück enthält.

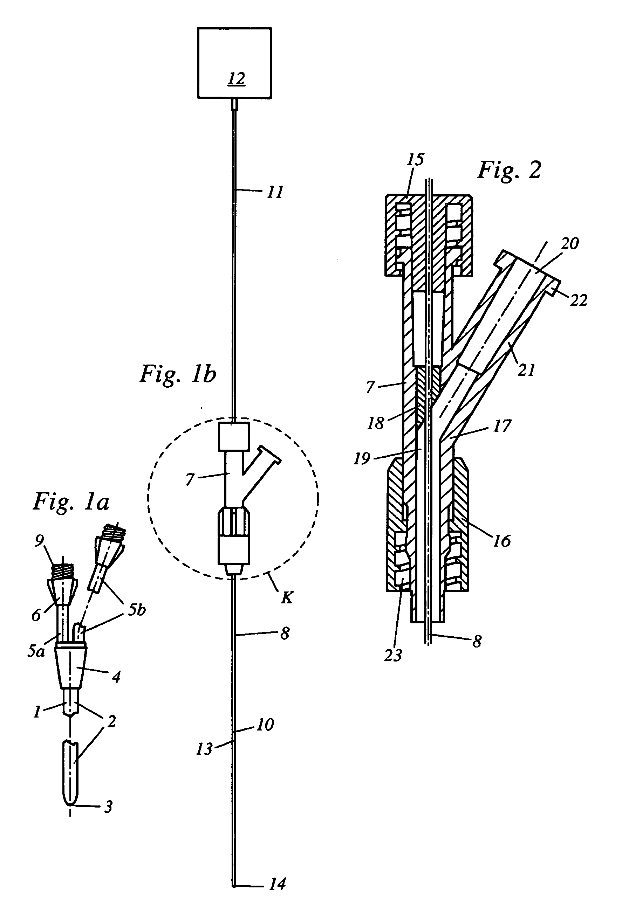

Der in Fig. 1a dargestellte zentralvenöse Multilumenkatheter 1 weist einen flexiblen, länglichen, zentralvenös applizierbaren, unterbrochen dargestellten Grundkörper 2 auf, in welchem mehrere Lumina ausgebildet sind, deren distale Öffnungen (nicht dargestellt) am distalen Ende 3 des Grundkörpers 2 bzw. in der Nähe des distalen Endes 3 des Grundkörpers 2 angeordnet sind. Proximal verlaufen die Lumina oberhalb einer Verzweigung 4 in mehreren Fortsätzen 5a, 5b weiter, wobei Fortsatz 5b unterbrochen dargestellt ist. Das Faseroptiklumen (nicht sichtbar), dessen Innendurchmesser deutlich größer ist als der Außendurchmesser des distalen Teils 10 der Faseroptiksonde 8, verläuft vom distalen Ende 3 des Katheter-Grundkörpers 2 durch diesen hindurch und weiter durch den Fortsatz 5a zu einem Gegenstück 6 für das Anschlußstück 7 der Faseroptiksonde 8. Über den Fortsatz 5a und der Verzweigung 4 ist das Gegenstück 6 fest mit dem Grundkörper 2 verbunden. Das Gegenstück 6 weist ein Außengewinde 9 auf, über welches das Anschlußstück 7 nach Einführen des distalen Teils 10 der Faseroptiksonde 8 in das Faseroptiklumen formschlüssig angeschlossen werden kann.

Die in Fig. 1b dargestellte Faseroptiksonde 8 eignet sich zur gleichzeitigen, gegenseitig unbeeinflußten Messung der zentralvenösen Sauerstoffsättigung und der lokalen Konzentration injizierten Indocyaningrüns. Hierzu ist sie über proximal in einem Kabel 11 verlaufende optische Fasern an eine Lichtquellen- und Meßvorrichtung 12 angeschlossen, welche zur gleichzeitigen Aussendung und Messung von Strahlung zweier Meß- und einer Referenzwellenlänge ausgebildet ist und über eine Auswerteinheit verfügt. Bei der ersten Meßwellenlänge von 660 nm unterscheiden sich die Reflexionseigenschaften von sauerstoffbeladenem und sauerstofffreiem Hämoglobin im Blut besonders stark. Bei der zweiten Meßwellenlänge von 805 nm liegt das Absorptionsmaximum von Indocyaningrün. Die Referenzwellenlänge beträgt 880 nm, da hier kein wesentlicher Unterschied der Reflexion zwischen sauerstoffgeladenem und sauerstofffreiem Hämoglobin im Blut existiert, und Indocyaningrün denselben molaren Absorptions- bzw. Reflexionskoeffizienten aufweist, wie oxygeniertes Hämoglobin. Die Berechnung der zentralvenösen Sauerstoffsättigung erfolgt anhand des Verhältnisses zwischen Reflexionsmessungen bei der ersten Meßwellenlänge und der Referenzwellenlänge, und die Berechnung der zentralvenösen Konzentration von Indocynaingrün anhand des Verhältnisses zwischen Reflexionsmessungen bei der zweiten Meßwellenlänge und der Referenzwellenlänge anhand aus Literatur und Technik bekannter, computerimplementierter Algorithmen.

Distal verlaufen die optischen Fasern in einem dünnen, flexiblen Schaft 13, welcher in der Nähe seines abgerundeten distalen Endes 14 mit einem antithrombogenem Überzug versehen ist. Die Länge des distalen Teils 10 ist auf die Länge des Faseroptiklumen des Multilumenkatheters 1 abgestimmt.

Mit der faseroptischen Sonde 8 ist das Anschlußstück 7 fest verklebt, welches in Fig. 2 im Querschnitt dargestellt ist. Das Anschlußstück 7 besteht aus vier miteinander verklebten Teilen 15, 16, 17, 18, von welchen zumindest das Abschlußteil 15 mit der faseroptischen Sonde 8 verklebt ist. Das Führungsteil 18 stabilisiert die Sonde 8 im Anschlußstück 7. Das Gewindeteil 16 weist ein Innengewinde 23 auf, über welches das Anschlußstück 7 an dem Gegenstück 6 des Multilumenkatheters 1 angeschlossen werden kann. Im angeschlossenen Zustand setzt sich das Faseroptiklumen im Inneren 19 des Y-Teils 17 des Anschlußstücks 7 fort. Das Faseroptiklumen ist dann proximal mittels des Abschlußteils 15 dicht abgeschlossen.

Das Innere 19 des Y-Teils 17 setzt sich in dem Spülkanal 20 fort, welcher durch den Spülstutzen 21 verläuft, welcher dem Y-Teil 17 angeformt ist und in einem Flansch 22 endet. Über den Flansch 22 ist der Spülkanal 20 verschließbar; zudem ist hier eine Spülvorrichtung (nicht dargestellt) anschließbar, so daß das Faseroptiklumen über das Innere 19 des Y-Teils 17 gespült werden kann.

## Patentansprüche

1. Kathetersystem zur kontinuierlichen Messung der zentralvenösen Sauerstoffsättigung und/oder der Messung der lokalen Konzentration injizierten Indocyaningrüns, aufweisend
einen flexiblen, länglichen, zentralvenös applizierbaren Grundkörper (2),
eine faseroptische Sonde (8) zur Vornahme reflexionsoximetrischer Messungen,
ein Faseroptiklumen zur Aufnahme der faseroptischen Sonde (8) und Befestigungsmittel zur Vermeidung einer Längsverschiebung der faseroptischen Sonde (8) relativ zu dem Faseroptiklumen, wobei die Befestigungsmittel lösbar sind, um eine Längsverschiebung der faseroptischen Sonde (8) relativ zu dem Faseroptiklumen zur Entnahme der faseroptischen Sonde (8) zuzulassen,
**dadurch gekennzeichnet,**
**daß** die Befestigungsmittel ein fest mit der faseroptischen Sonde (8) verbundenes Anschlußstück (7) und ein fest mit dem zentralvenös applizierbaren Grundkörper (2) verbundenes Gegenstück (6) aufweisen, welche aneinander anschließbar sind, und daß im angeschlossenen Zustand der Abstand zwischen distalem Ende (3) des zentralvenös applizierbaren Grundkörpers (2) und distalem Ende (14) der faseroptischen Sonde (14) durch die festen Verbindungen zwischen Sonde und Anschlußstück sowie Grundkörper und Gegenstück auf einen vorbestimmten Wert eingestellt ist.

2. Kathetersystem gemäß Anspruch 1, **dadurch gekennzeichnet, daß** das Faseroptiklumen spülbar ist, wenn das Anschlußstück (7) an dem Gegenstück (6) angeschlossen ist.

3. Kathetersystem gemäß Anspruch 2, **dadurch gekennzeichnet, daß** das Anschlußstück (7) einen Spülansatz (21) zum Anschluß einer Spülvorrichtung aufweist.

4. Kathetersystem gemäß einem der vorangehenden Ansprüche, **dadurch gekennzeichnet, daß** der vorbestimmte Wert des Abstands zwischen distalem Ende (3) des zentralvenös applizierbaren Grundkörpers (2) und distalem Ende (14) der faseroptischen Sonde zwischen 10 und 40 mm beträgt, wobei das distale Ende (14) der faseroptischen Sonde über das distale Ende des zentralvenös applizierbaren Grundkörpers (3) hinausragt.

5. Kathetersystem gemäß einem der Ansprüche 1 bis 3, **dadurch gekennzeichnet, daß** der vorbestimmte Wert des Abstands zwischen distalem Ende (3) des zentralvenös applizierbaren Grundkörpers (2) und distalem Ende der faseroptischen Sonde (8) so gewählt ist, daß das distale Ende (14) der faseroptischen Sonde (8) mit dem Grundkörper (2) bündig verläuft.

6. Kathetersystem gemäß einem der vorangehenden Ansprüche, **dadurch gekennzeichnet, daß** die faseroptischen Sonde (8) einen dünnen, flexiblen Schaft (13) aus bio- und hämokompatiblem Material sowie mindestens eine afferente und mindestens eine efferente optische Faser aufweist.

7. Kathetersystem gemäß einem der vorangehenden Ansprüche, **dadurch gekennzeichnet, daß** die faseroptischen Sonde (8) im Bereich möglichen Blutkontakts einen antithrombogenen Überzug aufweist.

8. Kathetersystem gemäß einem der vorangehenden Ansprüche, **dadurch gekennzeichnet, daß** es Einführungshilfsmittel zur zentralvenösen Applikation des Grundkörpers aufweist.

9. Kathetersystem gemäß Anspruch 8, **dadurch gekennzeichnet, daß** die Einführungshilfsmittel einen Führungsdraht aufweisen.

10. Kathetersystem gemäß Anspruch 9, **dadurch gekennzeichnet, daß** die Einführungshilfsmittel ferner eine zur Einführung des Führungsdrahtes geeignete Punktionskanüle aufweisen.

11. Kathetersystem gemäß Anspruch 10, **dadurch gekennzeichnet, daß** die Einführungshilfsmittel ferner einen auf den Durchmesser des Führungsdrahtes abgestimmten, sich verjüngenden Dilatator aufweisen, dessen Schaftdurchmesser mindestens, so groß ist, wie der Außendurchmesser des zentralvenös applizierbaren Grundkörpers (2).

12. Kathetersystem gemäß einem der Ansprüche 9 bis 11, **dadurch gekennzeichnnet, daß** das Kathetersystem ferner
ein parallel zum Faseroptiklumen verlaufendes distales Lumen und
einen sich verjüngenden Hohlmandrin aufweist, dessen Innendurchmesser auf den Führungsdraht und dessen Außendurchmesser auf den Innendurchmesser des distalen Lumens abgestimmt ist.

13. Kathetersystem gemäß einem der vorangehenden Ansprüche, **dadurch gekennzeichnet, daß** die faseroptische Sonde (8) zur gleichzeitigen, gegenseitig unbeeinflußten Messung der zentralvenösen Sauerstoffsättigung und der lokalen Konzentration injizierten Indocyaningrüns ausgebildet ist.

14. Kathetersystem gemäß Anspruch 13, **dadurch gekennzeichnet, daß** die faseroptische Sonde an eine Lichtquellen- und Meßvorrichtung (12) anschließbar ist, welche zur gleichzeitigen Aussendung und Messung von Strahlung zweier Meß- und einer Referenzwellenlänge ausgebildet ist.

15. Kathetersystem gemäß Anspruch 14, **dadurch gekennzeichnet, daß** die erste Meßwellenlänge bei 660 nm, die zweite Meßwellenlänge bei 805 nm, und die Referenzwellenlänge bei 880 nm liegt.

16. Kathetersystem gemäß einem der vorangehenden Ansprüche, **dadurch gekennzeichnet, daß** es zusätzlich eine Heizvorrichtung zur Aussendung von Wärmeimpulsen für die Vornahme transpulmonaler Messungen von Herz-/ Kreislaufgrößen aufweist.

## Claims

1. A catheter system for continuously measuring the centrovenous oxygen saturation and/or measuring the local concentration of injected indocyanine green, having
a flexible, elongated, centrovenously applicable basic body (2),
a fiber-optic probe (8) for taking reflecto-oximetric measurements,
a fiber-optic lumen for housing the fiber-optic probe (8) and
fastening means for preventing a longitudinal shift of the fiber-optic probe (8) relative to the fiber-optic lumen, the fastening means being detachable to enable a longitudinal shift of the fiber-optic probe (8) relative to the fiber-optic lumen for removal of the fiber-optic probe (8),
**characterized** therein
that the fastening means have a connecting piece (7) securely connected with the fiber-optic probe (8) and a counterpart securely connected with the centrovenously applicable basic body (2) which can be joined together, and that, when connected, the distance between the distal end (3) of the centrovenously applicable basic body (2) and the distal end (14) of the fiber-optic probe (14) is set at a predetermined value by the secure connections between probe and connecting piece as well as basic body and counterpart.

2. Catheter system according to claim 1, **characterized** therein that the fiber-optic lumen can be cleaned when the connecting piece (7) is attached to the counterpart (6).

3. Catheter systen according to claim 2, **characterized** therein that the connecting piece (7) has a cleansing attachment (21) for connection to a cleansing device.

4. Catheter system according to one of the preceding claims, **characterized** therein that the predetermined value of the distance between distal end (3) of the centrovenously applicable basic body (2) and the distal end (14) of the fiber-optic probe is between 10 and 40 mm, the distal end (14) of the fiber-optic probe protruding beyond the distal end of the centrovenously applicable basic body (3).

5. Catheter system according to any of claims 1 to 3, **characterized** therein that the predetermined value of the distance between the distal end (3) of the centrovenously applicable basic body (2) and the distal end of the fiber-optic probe (8) is selected in such a way that the distal end (14) of the fiber-optic probe (8) extends flush with the basic body (2).

6. Catheter system according to one of the preceding claims, **characterized** therein that the fiber-optic probe (8) has a thin, flexible shaft (13) consisting of a bio-compatible and hemocompatible material as well as at least one afferent and at least one efferent optical fiber.

7. Catheter system according to one of the preceding claims, **characterized** therein that the fiber-optic probe (8) has an antithrombogenic cover in the area of a possible contact with blood.

8. Catheter system according to one of the preceding claims, **characterized** therein that it has insertion aids for the centrovenous application of the basic body.

9. Catheter system according to claim 8, **characterized** therein that the insertion aids have a guide wire.

10. Catheter system according to claim 9, **characterized** therein that the insertion aids also have a puncture cannula suitable for inserting the guide wire.

11. Catheter system according to claim 10, **characterized** therein that the insertion aids also have a tapered dilator adapted to the diameter of the guide wire, whose shaft diameter is at least as large as the outside diameter of the centrovenously applicable basic body (2).

12. Catheter system according to any of the claims 9 to 11, **characterized** therein that the catheter system further has
distal lumen extending parallel to the fiber-optic lumen, and
a tapered hollow mandrel, the inside diameter of which is adapted to the guide wire and the outside diameter of which is adapted to the inside diameter of the distal lumen.

13. Catheter system according to one of the preceding claims, **characterized** therein that the fiber-optic probe (8) is made for the simultaneous, mutually unaffected measurement of the centrovenous oxygen saturation and the local concentration of injected indocyanine green.

14. Catheter system according to claim 13, **characterized** therein that the fiber-optic probe can be connected to a light source and measuring device (12) which is configured for the simultaneous emission and measurement of radiation of two measuring wavelengths and one reference wavelength.

15. Catheter system according to claim 14, **characterized** therein that the first measuring wavelength is at 660 nm, the second measuring wavelength at 805 nm, and the reference wavelength at 880 nm.

16. Catheter system according to one of the preceding claims, **characterized** therein that it has, in addition, a heating device for emitting heat impulses for taking transpulmonary measurements of heart/circulation values.

## Revendications

1. Système de cathéter pour la mesure continue de la saturation en oxygène dans une veine centrale et pour la mesure de la concentration locale de vert d'indocyanine injecté, présentant
un corps de base (2) flexible, allongé, applicable dans une veine centrale, une sonde à fibre optique (8) pour effectuer des mesures d'oxymétrie de réflexion, une lumière à fibres optiques pour le logement de la sonde à fibre optique (8) et des moyens de fixation pour éviter un déplacement longitudinal de la sonde à fibre optique (8) par rapport à la lumière à fibres optiques, les moyens de fixation étant amovibles afin d'autoriser un déplacement longitudinal de la sonde à fibre optique (8) par rapport à la lumière à fibres optiques pour l'enlèvement de la sonde à fibre optique (8),
**caractérisé par le fait que**
les moyens de fixation présentent un élément de raccordement (7) relié de façon fixe à la sonde à fibre optique (8) et un élément antagoniste (6) relié de façon fixe au corps de base (2) applicable dans une veine centrale, lesquels éléments peuvent être raccordés entre eux, et **par le fait que**, dans l'état raccordé, la distance entre l'extrémité distale (3) du corps de base (2) applicable dans la veine centrale et l'extrémité distale (14) de la sonde à fibre optique (8) est réglée à une valeur prédéfinie par les liaisons fixes entre la sonde et l'élément de raccordement ainsi qu'entre le corps de base et l'élément antagoniste.

2. Système de cathéter selon la revendication 1, **caractérisé par le fait que** la lumière à fibres optiques est lavable, lorsque l'élément de raccordement (7) est raccordé à l'élément antagoniste (6).

3. Système de cathéter selon la revendication 2, **caractérisé par le fait que** l'élément de raccordement (7) présente une embase de lavage (21) pour le raccordement d'un dispositif de lavage.

4. Système de cathéter selon l'une quelconque des revendications précédentes, **caractérisé par le fait que** la valeur prédéfinie de la distance entre l'extrémité distale (3) du corps de base (2) applicable dans une veine centrale et l'extrémité distale (14) de la sonde à fibre optique se situe entre 10 et 40 mm, l'extrémité distale (14) de la sonde à fibre optique dépassant de l'extrémité distale du corps de base (3) applicable dans une veine centrale.

5. Système de cathéter selon l'une quelconque des revendications 1 à 3, **caractérisé par le fait que** la valeur prédéfinie de la distance entre l'extrémité distale (3) du corps de base (2) applicable dans une veine centrale et l'extrémité distale de la sonde à fibre optique (8) est choisie de telle sorte que l'extrémité distale (14) de la sonde à fibre optique (8) arrive au niveau du corps de base (2).

6. Système de cathéter selon l'une des revendications précédentes, **caractérisé par le fait que** la sonde à fibre optique (8) présente une tige (13) mince et souple à base de matériau biocompatible et hémocompatible ainsi qu'au moins une fibre optique afférente et au moins une fibre optique efférente.

7. Système de cathéter selon l'une des revendications précédentes, **caractérisé par le fait que** la sonde à fibre optique (8) présente un revêtement anti-thrombogène dans la zone de contact possible avec le sang.

8. Système de cathéter selon l'une des revendications précédentes, **caractérisé par le fait qu'**il présente des moyens auxiliaires d'introduction pour l'application dans une veine centrale du corps de base.

9. Système de cathéter selon la revendication 8, **caractérisé par le fait que** les moyens auxiliaires d'introduction présentent un fil de guidage.

10. Système de cathéter selon la revendication 9, **caractérisé par le fait que** les moyens auxiliaires d'introduction présentent également une canule de ponction appropriée pour l'introduction du fil de guidage.

11. Système de cathéter selon la revendication 10, **caractérisé par le fait que** les moyens auxiliaires d'introduction présentent également un dilatateur adapté au diamètre du fil de guidage et se rétrécissant, dont le diamètre de tige est au moins aussi grand que le diamètre extérieur du corps de base (2) applicable dans une veine centrale.

12. Système de cathéter selon l'une des revendications 9 à 11, **caractérisé par le fait que** le système de cathéter présente également une lumière distale agencée parallèlement à la lumière à fibres optiques et un mandrin creux se rétrécissant, dont le diamètre intérieur est adapté au fil de guidage et dont le diamètre extérieur est adapté au diamètre intérieur de la lumière distale.

13. Système de cathéter selon l'une des revendications précédentes, **caractérisé par le fait que** la sonde à fibre optique (8) est conçue pour la mesure simultanée et non influencée de façon réciproque de la saturation en oxygène dans une veine centrale et de la concentration locale de vert d'indocyanine injecté.

14. Système de cathéter selon la revendication 13, **caractérisé par le fait que** la sonde à fibre optique est apte à être raccordée à un dispositif de source lumineuse et de mesure (12), qui est conçu pour l'émission et la mesure simultanées de rayonnement de deux longueurs de mesure et d'une longueur d'onde de référence.

15. Système de cathéter selon la revendication 14, **caractérisé par le fait que** la première longueur d'onde de mesure est à 660 nm, la seconde longueur d'onde de mesure, à 805 nm, et la longueur d'onde de référence, à 880 nm.

16. Système de cathéter selon l'une des revendications précédentes, **caractérisé par le fait qu'**il présente en supplément un dispositif de chauffage pour l'envoi d'impulsions de chaleur pour effectuer des mesures transpulmonaires de grandeurs cardiaques et circulatoires.
